# EUROPEAN PATENT SPECIFICATION

(11) **EP 2 606 125 B1**
(45) Date of publication and mention of the grant of the patent: **22.11.2017**
(21) Application number: 11818884.6
(22) Date of filing: 22.08.2011
(51) Int. Cl.: C12N 5/0783, A61K 35/14, A61K 35/17, A61K 39/00

(54) **CELLS EXPRESSING TH1 CHARACTERISTICS AND CYTOLYTIC PROPERTIES**
ZELLEN ZUR EXPRESSION VON TH1-MERKMALEN UND ZYTOLYTISCHEN EIGENSCHAFTEN
CELLULES EXPRIMANT LES CARACTÉRISTIQUES ET LES PROPRIÉTÉS CYTOLYTIQUES DE THL

(30) Priority: 09.06.2011 US 201161495055 P; 20.08.2010 US 401881 P
(43) Date of publication of application: 26.06.2013
(73) Proprietor: Immunovative Therapies, Ltd., Jerusalem 96951 (IL)
(72) Inventor: HAR-NOY, Michael, 94817 Jerusalem (IL)
(74) Representative: Wilson, Justin Scott
(86) International application number: PCT/US2011/048578
(87) International publication number: WO 2012/024666

(56) References cited:
- US-B2- 7 425 592
- US-B2- 7 435 592
- C. JURSIK ET AL: "Large-Scale Production and Characterization of Novel CD4+ Cytotoxic T Cells with Broad Tumor Specificity for Immunotherapy", MOLECULAR CANCER RESEARCH, vol. 7, no. 3, 10 March 2009 (2009-03-10), pages 339-353, XP055101327, ISSN: 1541-7786, DOI: 10.1158/1541-7786.MCR-07-2208
- HAMID ECHCHAKIR ET AL: "Cutaneous T Cell Lymphoma Reactive CD4+ Cytotoxic T Lymphocyte Clones Display a Th1 Cytokine Profile and Use a Fas-Independent Pathway for Specific Tumor Cell Lysis", JOURNAL OF INVESTIGATIVE DERMATOLOGY, vol. 115, no. 1, 1 July 2000 (2000-07-01), pages 74-80, XP055101319, ISSN: 0022-202X, DOI: 10.1046/j.1523-1747.2000.00995.x
- DAMIEN Z SOGHOIAN ET AL: "Cytolytic CD4 + T cells in viral immunity", EXPERT REVIEW OF VACCINES, vol. 9, no. 12, 1 December 2010 (2010-12-01), pages 1453-1463, XP055101069, ISSN: 1476-0584, DOI: 10.1586/erv.10.132
- S. A. QUEZADA ET AL: "Tumor-reactive CD4+ T cells develop cytotoxic activity and eradicate large established melanoma after transfer into lymphopenic hosts", JOURNAL OF EXPERIMENTAL MEDICINE, vol. 204, no. 1, 15 March 2010 (2010-03-15), pages 49-650, XP055101338, ISSN: 0022-1007, DOI: 10.1084/jem.20062056
- HAR-NOY ET AL.: 'Completely mismatched allogeneic CD3/CD28 cross-linked Th1 memory cells elicit anti-leukemia effects in unconditioned hosts without GVHD toxicity' LEUKEMIA RESEARCH vol. 32, 18 June 2008, pages 1903 - 1913
- BROWN ET AL.: 'IL-2 and antigen dose differentially regulate perforin- and FasL-mediated cytolytic activity in antigen specific CD4+ T cells' CELLULAR IMMUNOLOGY vol. 257, 31 March 2009, pages 69 - 79
- HAR-NOY ET AL.: 'Allogeneic CD3/CD28 cross-linked Th1 memory cells provide potent adjuvant effects for active immunotherapy of leukemia/lymphoma' LEUKEMIA RESEARCH vol. 33, 01 October 2008, pages 525 - 538

## Description

### BACKGROUND

Therapeutic cancer vaccination is a type of immunotherapy. Immunotherapy is a new treatment modality that is emerging to join chemotherapy, radiation and surgery as a class of drugs for treatment of cancer. Immunotherapy methods seek to harness the power of the immune system in order to treat diseases. Therapeutic vaccination is a type of immunotherapy that is potentially curative therapy against existing tumors, viruses and bacterial pathogens. Therapeutic vaccines generally consist of an antigen source derived from the target disease and an adjuvant designed to enhance the desired immune response. In some therapeutic vaccine protocols, living immune cells, either derived from the host (autologous) or from a donor (allogeneic), are components of therapeutic vaccines. Autologous and allogeneic living immune cells, such as dendritic cells, NK cells and T-cells, are also used in immunotherapy protocols for the treatment of cancer.

Lymphocytes are part of the vertebrate immune system and include large granular lymphocytes and small lymphocytes. Large granular lymphocytes include natural killer cells (NK cells). Small lymphocytes consist of T-cells and B cells. NK cells are part of the innate immune system and play a major role in defending an animal from tumors and virally infected cells. NK cells can distinguish infected and tumor cells from uninfected cells through the multihistocompatibility complex (MHC) class I surface molecules. NK cells are activated in response to cytokines and upon activation release cytotoxic granules such as perforin and granzyme B that destroy the infected cells or tumor cells. NK cells are characterized by the cell surface markers CD16+ and CD56+, but do not express CD4.

T-cells and B-cells are part of the adaptive immune response and recognize non-self antigens. B-cells respond to non-self antigen by antibody production. Different types of T-cells respond to the non-self antigens in different ways. Cytotoxic T-cells (CTL) are characterized by the markers CD3+, CD8+ and TCRαβ, and do not express CD4. Tumor-specific CD8+ CTLs are mainly responsible for tumor elimination. Moreover, CTLs can specifically recognize tumor cells and do not attack normal cells of the same tissue. CTLs produce toxic granules that contain powerful enzymes including gramzyme B and perforin that induce death of diseased or cancerous cells.

T helper cells are characterized by the cell surface markers CD3+, CD4+ and TCRαβ. T-helper cells produce cytokines or other molecules that direct the immune response through other cells or molecules. T-helper cells are not known to directly mediate killing of cells and thus do not normally contain cytoxic granules such as granzyme B or perforin. T-helper cells are sub-categorized into two types: T-helper type 1 (Th1) and the T-helper type 2 (Th2). Th1 cells mediate cellular immunity and are critical for immune-mediated tumor eradication, whereas Th2 cells mediate a humoral or antibody immunity. Th1 and Th2 cells are counter-regulatory, increased Th1 cells down regulate Th2 cells and vice versa.

A variety of immunotherapy methods and compositions have been developed in order to enhance or suppress the immune response in patients. Cell therapy methods often involve *ex-vivo* manipulations such as proliferation, differentiation and/or activation of cells and the transfer of these cells to a patient as therapy.

Adoptive immunotherapy involves removing lymphocytes from the patient, boosting their anti-cancer activity ex-vivo, growing the cells to large clinically relevant numbers, and then returning the cells to the patient:

Initial experiments in adoptive immunotherapy involved removing lymphocytes from the blood of a patient and growing them in the presence of the lymphokine interleukin-2 (IL-2), an immune stimulator. The cells were then returned to the patient. These lymphocytes were called lymphokine-activated killer (LAK) cells.

A stronger response against tumor cells was obtained using lymphocytes isolated from the tumor itself. These tumor-infiltrating lymphocytes (TILs) are grown in the presence of IL-2 and returned to the body to attack the tumor.

Adoptive immunotherapy is a form of immunotherapy where ex vivo processed cells are introduced into the body. Pre-clinical studies suggested anti-tumor activity could be enhanced by using interleukin-2 together with ex vivo activated and expanded autologous lymphocytes. Also, the first objective responses with high-dose bolus interleukin-2 therapy were noted in patients receiving interleukin-2 together with LAK cells prepared through in vitro activation of autologous peripheral blood lymphocytes that were harvested by lymphopheresis, and initially, it appeared that the combination of interleukin-2/LAK was more active than interleukin-2 alone. IL-2 is a cytokine produced by Th1 helper cells. Interest in the use of adoptive immunotherapy declined after clinical studies failed to demonstrate that the addition of activated LAK or TIL cells with IL-2 is any more effective than IL-2 alone.

CD4+ T (Th) cells are crucial for the activation and regulation of most aspects of the host defense against infections and for adequate function of cytotoxic CD8+ lymphocytes (CTL). Much of the research in tumor immunology has been focused on naturally occurring autologous tumor-specific T cells that are predominantly CD8+ and can be isolated from melanoma and some other tumors. These cells can be expanded in vitro and reinfused. This type of adoptive immunotherapy has resulted in objective tumor responses and long-term survival in some patients whose disease is refractory to other interventions. While there is an extensive clinical experience using CD8+ cytotoxic T cells as adoptive immunotherapy, poor clinical responses underscore the need to improve these therapies in order to achieve tumor rejection in higher percentages of patients.

CD8+ lymphocytes can fail to maintain functionality in vivo in large part because of the absence of CD4+ T cell help. In vitro, CD8+ cells release large quantities of interferon-γ (IFN-γ) upon exposure to MHC-compatible cell lines and lyse autologous antigen-positive and MHC class I-positive tumors. However, the genetic instability of tumor cells frequently leads to losses in the ability to process and present endogenous antigens rendering tumors inherently unreliable targets for CD8+ cytolytic T cells. Furthermore, CD8+ T cells appear to lack the intrinsic ability to orchestrate a broad antitumor response that seems inherent in some CD4+ T cell subsets.

While immunotherapies with adoptive transfer of CTL have demonstrated promise in many animal models, the translation of these results to humans has proven to be difficult and elusive. CD4+ cells, especially of the Th1 subtype, would be an attractive addition to the available immunotherapy armamentarium to provide natural "help" to adoptively transferred CD8+ killer cells. However, there are significant barriers in working with naturally occurring tumor-specific CD4+ cells. The genetic diversity of the class II HLA associated with CD4+ helper cells in any given population of patients is much more complex than in the case of class I HLA found in CD8+ cytolytic (killer) T-cells, making identification of epitopes and TCRs more problematic. Moreover, CD4+ T-cells expand in vitro less well than CD8+ cells and the culture conditions significantly affect their characteristics. Finally, there is a paucity of realistic animal models based on tumor-specific CD4+ cells. These factors have limited translational research using CD4+ T cells and limited their clinical use.

Disclosed herein is a method for producing clinically-relevant numbers of CD4+ cells that also contain the cytolytic granules granzyme B and perforin and function both as Th1 cells (produce IFN-gamma and not IL-4) and have NK-like activity (recognize and kill tumor cells and not normal cells) for use in therapeutic cancer vaccine and adoptive immune cell therapy protocols.

### SUMMARY

Cell-based therapeutic cancer vaccines (cellular immunotherapy) hold promise as a new minimally toxic approach for treatment of cancer. The promise of this approach has been demonstrated in animal models, but it has been difficult to translate this promise to the clinic (Bodey, Bodey et al. 2000). The recent FDA approval of the first cellular immunotherapy drug, Provenge® (sipuleucel-T), an autologous dendritic cell immunotherapy for the treatment of asymptomatic or minimally symptomatic metastatic castrate resistant prostate cancer (Kantoff, Higano et al.), has renewed enthusiasm to develop improved cell therapy products for clinical application.

Since it has proven difficult to successfully translate the immune-mediated anti-tumor mechanisms of cellular immunotherapy strategies developed in animals, we instead concentrated on translating an anti-tumor immune mechanism already known to elicit significant clinical anti-tumor effects. Arguably, the most powerful and most effective immune-mediated anti-tumor mechanism ever described in humans is the graft vs. tumor (GVT) effect that occurs after non-myeloablative conditioning and transplant of HLA-matched stem cells (Barrett and Childs 2000; Childs 2000; Resnick, Shapira et al. 2005). Unfortunately, these GVT effects are often accompanied by chronic graft-versus-host-disease (GVHD) toxicity. Chronic GVHD is associated with significant morbidity and is the leading cause for late mortality after allogeneic hematopoietic stem cell transplantation (Lee, Klein et al. 2002), significantly limiting the clinical application of the GVT mechanism.

Separation of the beneficial GVT effects from the detrimental GVHD effects is difficult because the effects are interrelated and proportional, in that manipulations that inhibit GVHD also decrease the GVT effect (Li, Giver et al. 2009). By analyzing the known immune-mediated mechanisms of the inter-related GVT and GVHD effects, it was hypothesized that it might be possible to develop an allogeneic cellular immunotherapy that instead of separating the GVT/GVHD effects would maintain the interrelationship of the immune mechanisms by reversing the direction of the effects from graft-to-host to host-to-graft (Har-Noy and Slavin 2008). Successful reversal of the direction of the effects would result in a host vs. tumor (HVT) effect coupled to a non-toxic host vs. graft (HVG) rejection effect.

It was hypothesized that intentionally mis-matched, activated, allogeneic Th1 cells that expressed high density CD40L and produced high amounts of interferon (IFN)-γ would be capable of eliciting coupled HVT/HVG effects. This hypothesis was tested in an animal model of chemotherapy-refractory B-cell leukemia/lymphoma (Har-Noy, Zeira et al. 2008). These studies demonstrated that significant HVT/HVG activity could be elicited by ex-vivo differentiated allogeneic Th1 cells only when the Th1 cells were activated and administered attached to anti-CD3/anti-CD28-coated microbeads. Additional studies indicated that these allogeneic CD3/CD28-crosslinked Th1 cells had both immunomodulatory effects, capable of switching systemic dominant Th2 immunity to dominant Th1 immunity; and, adjuvant effects for promoting development of Th1-specific immunity and dysregulation of tumor immunoavoidance mechanisms (Har-Noy, Zeira et al. 2009). In view of these data, human version of Th1 allografts were investigated for the possibility of translating this experimental approach to the clinic.

There were significant regulatory and logistical concerns associated with the ex vivo differentiation and expansion of allogeneic Th1 cells that were necessary to be overcome in order to use these cells for human clinical investigations. One such issue was that the mouse version of allogeneic Th1 cells required the use of exogeneous cytokines, IL-2, IL-12 and anti-IL-4 mAb to cause the ex-vivo differentiation of mouse Th1 cells. The current invention discloses the successful development of a GMP-compliant process for the production of human allogeneic Th1 cells without the use of exogenous cytokines resulting in consistent batch-to-batch identity and functional characteristics for classification as a human biological drug. Upon characterization of the resulting allograft cells, the human production process resulted in a unexpected finding. The allogeneic Th1 cells generated by the methods described herein not only developed Th1 characteristics, but also developed NK-like characteristics. An allograft with a combination of Th1 and NK-like activity is unique and is not described in normal blood circulation. The methods disclosed herein involve the ex-vivo culture of purified, resting (CD25-), CD4+ T-cells with mixed memory and naive phenotype (CD45RA and CD45RO, respectively). The cells are activated multiple times with immobilized anti-CD3/anti-CD28 mAbs in the absence of exogenous cytokines. The cells expand 25-100-fold after 9-days and differentiate into CD4+, CD45RO+, CD25+, CD62^{hi} cells. This intermediate cell can be stored in liquid nitrogen for years. When needed for patient administration, the precursor cells are incubated for 4h with CD3/CD28 mAb coated microbeads. During this final 4h incubation, the precursor cells further differentiate to decrease expression of CD62L and increase expression of CD40L and CD25. The final CD4+, CD45RO+, CD40L^{hi}, CD62L^{lo}, CD25+ cells produce >1000pg/10⁶ cells of IFN-gamma and <50pg/10⁶ cells of IL-4 (Th1 phenotype) and express cytolytic granules of granzyme B and perforin and exhibit NK-like activity with the ability to lyse tumor cells and not normal cells.

### BRIEF DESCRIPTION OF THE DRAWINGS

Fig. 1A and Fig. 1B are flow cytometry plots from immunostaining of CD4 surface antigen demonstrating the cell phenotype before and after CD4+ selection, respectively.
Fig. 2A is a series of flow cytometry plots demonstrating the phenotype of source cells having as resting CD4 naive (CD4, CD45RA) cells.
Fig. 2B is a series of flow cytometry plots demonstrating the change in phenotype after culture to activated CD4 memory cells (CD4, CD45RO and CD25).
Fig. 3 is a flow cytometry plot demonstrating the shift in CD62L expression from high to low inCAC before activation and CFB after activation.
Figs. 4A-4C are plots of the CD40L expression pattern in CD4+ cells, CAC and CFB, respectively demonstrating increased expression of CD40L in CFB.
Fig. 5 is a graph of the direct killing assay and shows CFB can kill cells from the tumor cell line, ARH77.
Fig. 6A is a plot of expression of Granzyme B by CAC.
Fig. 6B is a plot of expression of Granzyme B by CFB.
Fig. 6C is a picture of a western blot showing detection of Perforin in CFB.
Fig. 7 is a graph of perforin-Granzyme B activity with and without EGTA.

### DETAILED DESCRIPTION OF ILLUSTRATIVE EMBODIMENTS

This disclosure relates to compositions that include cells with unique characteristics. The composition includes cells of a cell type having both Th1 helper cell properties and cytolytic granules and NK activity. CD4+ T-cells with the combination of Th1 characteristics and NK characteristics can provide substantial therapeutic capabilities for patients having a variety of diseases. Th1 characteristics can include, for example, production of IFN-gamma without production of IL-4. NK characteristics can include killing of tumor cells but not normal cells utilizing expression of Granzyme B and perforin. In particular, the invention provides a composition comprising activated CD4+ Th1/killer cells having Th1 characteristics and direct cytolytic activity against a tumour cell line as defined by claim 1 hereinafter.

Herein disclosed are methods to cause the differentiation and expansion of this novel cell type to clinically-relevant numbers for adoptive immunotherapy. Methods for deriving the novel cells can include isolating T-cells, specifically CD4+ T-cells from the peripheral blood or other sources of T-cells. These CD4+ T-cells can be differentiated and expanded according to the methods described herein to clinically-relevant numbers and used for immunotherapy. Surprisingly, the CD4+ T-cells prepared according to these methods can exhibit cytolytic activity similar to the cytolytic activity generally found in NK/CTL cells and also exhibit Th1 characteristics and activity.

Generally, T-helper cells do not directly exhibit cytolytic activity but produce cytokines and other factors that then elicit other cells such as NK cells and CTLs to inactivate or kill the diseased cells. The CD4+ T-cells in the present compositions can directly kill the diseased cells as well as produce cytokines to stimulate other cells such as NK cells that then carry out the cytolytic activity. The cytolytic activity of these CD4+ T-cells is mediated through Granzyme B and perforin resulting in the killing of the tumor or infected cells.

Cytolytic activity as referred to herein relates to direct killing by the effector cells of target tumor cells or cell lines, when they interact with the target cells. Direct killing can occur by the presence of only the effector and the target cells and without the presence of any other cell types.

The novel cell type described in the present invention will be referred to herein as Thl/killer cells. The Th1/killer cells can exhibit a combination of Th1 characteristics, killing of tumor cells and not normal cells and expression of NK activity through a mechanism of Granzyme B and perforin expression.

Clinically-relevant number of cells as referred to herein relates to sufficient number of the Th1/killer cells to elicit an anti-tumor effect either directly or indirectly. The number of Th1/killer cells administered generally is at least 1 x 10⁶ cells, preferably at least 1 x 10⁷ number of cells, and more preferable at least about 1 x 10⁸ number of cells in a single dose. Doses with greater amount of cells are also within the scope of the invention. Preferably sufficient amount of cells are produced from a single blood donor to produce enough doses for multiple patients. Clinically relevant numbers include enough cells for at least one patient, more preferably for up to 10 patients and most preferably for over 100 patients.

The compositions of the present invention can be administered to a patient having a variety of diseased cells. The patient can be animal, human or other mammals. The diseased cells can be cancerous cells or cells from infected tissue. Diseased cells may be infected with viruses and/or bacterial pathogens. The compositions may be administered to patients with solid tumors such as breast cancer, lung cancer, colon cancer, stomach cancer, pancreatic cancer and the like. The compositions may also be administered to patients with hematological malignancies (e.g., Chronic Lymphocytic Leukemia, Multiple Myeloma, and non-Hodgkin's lymphomas) or viral infectious diseases (e.g., hepatitis B or C, herpes, HIV) and other disorders.

The composition described herein is live cells. By live cells, it is meant that >70% of the cells are viable as determined by appropriate assay techniques such as trypan blue extrusion, MTT or bioluminescent detection of the ATP levels such that the cells are capable of *ex vivo* manipulations such as expansion, differentiation, and/or activation under appropriate conditions. The compositions, however, may include some inactivated cells, radiated cells and/or non-viable cells and non-living components.

The composition generally includes CD4+ cells first derived, for example, from blood of a donor. The donor can be without disease or normal. The CD4+ cells may be processed in a manner as described herein and then formulated for infusion into the same donor or into a related or unrelated recipient.

The CD4+ source cells are preferably purified from the peripheral blood. The CD4 cells can be purified in a variety of ways. Generally, the CD4+ cells are purified from the buffy coat of peripheral blood by positive selection. Positive selection of the CD4+ from buffy coat can be performed, for example, by using magnetic beads and columns obtained from Miltenyi Biotec (Auburn, CA). This can result in a cell composition that is greater than about 90 percent, preferably greater than about 95 percent, and more preferably greater than about 98 percent pure CD4+ cells.

The CD4+ cells isolated from the buffy coat and used as source material for production of Thl/killer cells can be characterized by expression of a number of cell surface markers. The population of CD4+ cells purified from the buffy coat of the peripheral blood can have a mixed population of CD45RA/CD45RO markers. The CD4+ T-cells are generally predominantly CD25- . Preferably the source CD4+ cells are mostly naive (express CD45RA and not CD45RO). Source CD4+ T-cells can also be evaluated for CD62L expression and CD40L expression. Generally, the source CD4+ T-cells express very low amounts of CD40L. Expression of CD62L can be a bimodal (high and low) mean fluorescent intensity (MFI) peak. Source CD4+ cells preferably have a high CD62L expression.

The source CD4+ T-cells can be processed to differentiate into Th1/killer cells by multiple activation steps. Preferably the CD4+ cells are activated every three days for 9 days. These CD4+ cells are activated through cross-linking of CD3 and CD28 cell surface molecules. Cross-linking of CD3 and CD28 can be accomplished by adding immobilized anti-CD3 and CD28 monoclonal antibodies. Generally, the Thl/killer cells are obtained by differentiation and expansion in cell cultures. The multiply activated CD4+ cells can be harvested from the cell culture and stored frozen in liquid nitrogen. Prior to administration to a patient, the cells are activated a final time. The activating agent is left associated with the cells. The cells with the activating agent attached is formulated in a device, such as a syringe, suitable for infusion or injection. "Activation" as used herein refers to both binding of the cell surface molecules by agents such as monoclonal antibodies and cross-linking of these agents by cross-linking agents. This type of activation is, for example, described in U.S. Patent No. 7,435,592 and U.S. Patent No. 7,402,431.

The CD4+ T-cells may be activated in a variety of ways including by the use of immobilized monoclonal antibodies specific for T-cell surface molecules. Suitable activated T-cells are, for example, described in U.S. Patent No. 7,435,592. The cells preferably have cell surface moieties that are bound by monoclonal antibodies or other binding agents that are then cross-linked. These monoclonal antibodies and/or binding agents are preferably cross-linked by an agent, for example, immobilized on a solid surface in order to activate the T-cells. These are referred to herein as cells activated in culture (CAC). These *ex vivo* prepared CAC can be frozen for future use or formulated for infusion. The CAC, for example, may be aliquoted and frozen in the gas phase of Liquid Nitrogen tanks. The CAC are capable of consistently meeting predefined identity and functional release criteria as described below. In process and final quality control tests are established to assure sterility and endotoxin-free status.

In one exemplary embodiment, the CD4+ T-cells are cultured for 9 days in the presence of CD3/CD28 ClinEx Vivo Dynal Beads obtained from Invitrogen, Carlsbad, CA. The cells are grown in Life Cell Flasks (Baxter Scientific, Deerfield, IL) at 37°C and 5% CO₂ and re-stimulated with the beads at days 3 and 6 of culture. After 9 days in culture, the beads can be removed and the cells can be referred to as CAC. Other methods of culturing cells for activation are also within the scope of the invention.

In preferred embodiments, the *ex vivo* prepared CAC are stored frozen until needed for patient administration or other uses. Prior to administration to the patient, the CAC are thawed, if necessary, washed and reactivated in nutrient media by cross-linking of the cell surface binding moieties such as CD3 and CD28 as described, for example in U.S. Patent No. 7,402,431. The CAC, together with the activation and cross-linking agents, can then be washed and transferred to a non-nutritive buffer such as a formulation buffer. The reactivated cells in formulation buffer are referred to herein as cells in formulation buffer (CFB). CFB may also be referred to herein as Th1/killer cells. CFB and Th1/killer cells may be used interchangeably herein.

The Th1/killer cells can be packed in a syringe or other suitable containers and transferred to a point of care site, if needed. The Th1/killer cells can then be administered to the patient for therapeutic purposes. The Th1/killer cells may be administered intravenously, intraperitoneally, intradermally or by other suitable methods. Th1/killer cells, once transferred to non-nutritive buffer have a limited shelf life. Cells can be formulated at a density of at least about 10⁶ cells per ml, preferably at about 10⁷ cells per ml or higher. In some embodiments, the cells may be formulated at a density at about 10⁸ cells per ml or higher. The specific concentration of the cells may be determined by the specific use of the cells and the therapy protocol.

The composition may also include a number of other components in addition to the Th1/killer cells. These components can include, for example, agents that maintain the cells in the desired activation state. In one exemplary embodiment, the composition can include agents that maintain the T-cells in an activated state such as Dynabeads ClinExVivo™ described below in the Examples.

Generally, the cells are transferred to non-nutritive buffer that is appropriate for infusion into a patient. The cells can be in a variety of non-nutritive buffers. Non-nutritive buffer, as referred to herein, is any type of media, buffer or other liquid that lacks the appropriate components to support cellular proliferation and/or expansion. The non-nutritive buffers generally are isotonic, USP sterile, pyrogen-free and contain the appropriate components and/or buffering system to maintain live cells intact and are licensed for human parenteral use. In an exemplary embodiment, the non-nutritive buffer is a formulation buffer that is Plasmalyte A (Baxter Scientific, Deerfield, IL) with 1% human serum albumin. (McKesson, San Francisco, California)

In embodiments with Th1/killer cells, the activation signals for the cells are maintained even when the cells are transferred to the non-nutritive buffer. For example, in embodiments where the cells are activated by cross-linking the cell surface binding moieties, the cross-linking is preferably maintained in the non-nutritive buffer. The maintenance of the cross-linking during storage may be critical to restoring the functional characteristics of the composition after removal from storage.

The Th1/killer cells in the composition can exhibit a novel combination of functional characteristics. These functions include Th1 characteristics and cytolytic functions that are similar to NK functions. The Th1/killer cells generally produce IFN-gamma and have substantially diminished or no IL-4 production. Preferably, the IL-4 production is below 50pg/10⁶ cells and IFN-gamma production greater than 1000pg/10⁶ cells.

Additional functional characteristics of the Th1/killer cells can include for example, expression of functional molecules such as CD40L, FasL, perforin and granzymeB, co-stimulatory molecules 4-1BBL, CD28, CTLA4, and TNF-related activation-induced cytokine (TRANCE), TWEAK, PD-1, B7 family, adhesion molecules such as the integrins, the cadherins, and the selectins and secretion of a variety of cytokines and chemokines and expression of receptors for these cytokines and chemokines. The Thl/killer cells can produce and secrete large amounts of cytokines. The cytokines produced can be, for example, IFNa, GM-CSF and TNF-α, with only residual IL4 secretion

The Thl/killer cells described herein can also exhibit cytolytic activity. The cytolytic activity is similar to the characteristics of CTL/NK cells. Generally, the cytolytic activity of CTL/NK cells, as well as the Thl/killer cells, is specific for tumor or diseased cells but not normal cells. In other words, the Thl/killer cells can kill diseased cells but not normal cells. The cytolytic activity can occur through a variety of mechanisms. The cytolytic activity can occur, for example, through the Granzyme B-Perforin mechanism. The cytolytic activity may lead to destruction of the cells by cytolysis or apoptosis. The cytolytic activity of the Th1/killer cells can be blocked by EGTA. Concentration of EGTA at about 1mM, for example, can decrease the cytolytic activity of the activated Th1 cells.

Thl/killer cells of the present invention express the molecules Granzyme B and Perforin. Granzyme B and Perforin are generally not expressed in CD4+ T-cells, whether naive or after maturation by differentiation, activation and the like. The amount of Granzyme B and Perforin in the activated Th1 cells can vary. Generally, the amount of Granzyme B and Peforin expressed is sufficient to destroy diseased cells.

The present invention includes compositions that include Thl/killer cells. These Thl/killer cells can inactivate a variety of tumor cells including the myeloma tumor cell lines such as ARH77 cells. The inactivation of the diseased cells can be through a mechanism sensitive to EGTA. The effector cell (Th1/killer cells) to target cell (diseased cell) ratio can vary. The effector to target cell ratio can be at least 1:9, preferably at least 1:5 and more preferably at least 1:1.

The composition of the present invention can be used for destroying diseased cells. The diseased cells can be in a patient. The patient, for example, may have cancer or contain infected tissue due to infection by a virus. The patient may have a solid tumor or hematological malignancy. This use includes administering to the patient a composition that includes the Thl/killer cells described herein. The Th1/killer cells are preferably allogeneic to the patient. The allogeneic cells may be from one normal allogeneic donor. Alternatively, the allogeneic cells may be derived from multiple donors and combined into a therapeutic composition. Preferably, the histocompatibility mismatch between the patient and allogeneic cells is maximized. Some histocompatibility between the patient and the allogeneic cells can be tolerated and can still result in the desired effect.

The Th1/killer cells can be administered to the patient by a variety of routes including intradermally, intravenously, intraperitoneally and the like. The patient may be administered one dose or multiple doses. The number of cells administered to the patient can vary and may depend on the specific disease, the patient, the method of activation and other factors. Generally, the patient is administered at least 10⁷ of activated Th1 cells, preferably at least 10⁸ and more preferably at least about 10⁹ cells.

The composition of the present invention can also be used in treating a patient by administering a composition that includes Th1/killer cells. The patient may receive one or more doses of the Th1/killer cells. The amount and the frequency of the doses can vary and can depend on the specific disease. In preferred embodiments, the Th1/killer cells are allogeneic to the patient and administration of the Th1/killer cells to the patient induces a host versus tumor effect along with a host versus graft effect without graft versus host disease.

### EXAMPLES

**Materials:** PE-conjugated CD40L was purchased from Beckman Coulter, Brea, CA. 7-Amino-Actinomycin D (7-AAD) (1000x) was purchased from Cayman Chemical Co., Ann Arbor, MI. PlasmaLyte A was purchased from Baxter Scientific, Deerfield, IL. Human serum albumin (HSA) was purchased from McKesson, San Francisco, California. FcR Binding Inhibitor was purchased from eBioscience, San Diego, CA. Dynabeads ClinExVivo™ was purchased from Invitrogen, Carlsbad, CA.

**Preparation of Cells Activated in Culture**(CAC)**-** CD4+ T-cells were cultured for 9 days in the presence of CD3/CD28 ClinEx Vivo Dynal Beads obtained from Invitrogen. (location). The cells were grown in Life Cell Flasks (Baxter) at 37°C and 5% CO₂ and re-stimulated with the beads at days 3 and 6 of culture. After 9 days in culture, the beads can be removed and these cells are referred to as CAC.

**Preparation of Cells in Formulation Buffer** (CFB)-CAC were placed into cRPMI media for washing. Time was recorded to indicate the beginning of the formulation protocol. The cells in cRPMI media were centrifuged, the supernatant removed and the cells resuspended in cRPMI buffer. Cell viability was determined by using Trypan Blue assays. The total cell number and the concentration of live cells were used to determine the percentage of viable cells. If the sample had greater than 80 percent cell viability, then the procedure was continued for reactivation and formulation of cells.

The CAC cells were resuspended at a concentration of 1 x 10⁷ cells/ml. Reactivation was done at a live cell concentration of 1 x 10⁷ cells/ml. Reactivation was done in a 24 well plate, 6 well plate or a 75 cm³ flask depending on the volume. Dynabeads ClinExVivo™ CD3/CD28 were added to reactivate the cells and incubated at 36-38°C and 5% CO₂ for 4 hours.

After incubation for about 4 hours, then the cells were removed and transferred to a 50 ml. tube with final formulation buffer (FFB). FFB is PlasmaLyte A with 1% HSA. The reactivated cells were centrifuged, supernatant removed and resuspended in FFB. These are referred to as cells in formulation buffer (CFB).

CFB were resuspended in FFB at a concentration 10⁷ cells per ml. The CFB were resuspended for ID, IT or IV administration. 1ml of the cell suspension was added to a 3ml syringe as an ID formulation. IT and IV formulation were 3 ml and 5 ml, respectively. The syringes with the appropriate formulations were stored in refrigeration with an average temperature of about 4°C.

**Harvesting of samples after storage**-The cells and supernatant were collected at different time points. The time points were as follows: 0(initial); 2 hours at Room Temp (RT); 48 hours at 4°C; and 48 hours at 4°C followed by 2 hours RT.

At each time point, 100ul cell suspensions were collected and the cells were spun at 400g for 5 min at 4°C. The supernatant was then transferred to another tube for IFN-γ detection later using ELISA. The cells were resuspended in 150ul staining buffer for flow cytometry. In some experiments, the cells were resuspended in 100ul cRPMI medium and cultured in the incubator at 37°C for 24 h with 5% CO₂. The supernatant was taken after 24 h incubation and the IFN-γ was detected by ELISA.

**Flow cytometry (CD40L and 7-AAD)-** 50ul cell suspension were transferred from above (150 ul) into 3 eppendorf tubes, labeled as unstained, CD40L and 7-AAD, respectively. The unstained tube was incubated on ice for 20 min. For the CD40L tube, the cells were preincubated with FcR Binding inhibitor according to the instructions of the manufacturer for 20 minutes on ice. Then 40ul staining buffer (PBS+1%FBS) and 10ul PE-CD40L antibody was added into the cell suspension and incubated for additional 20 min on ice in the dark.

Cell viability was tested by flow cytometry of 7-AAD. 7-AAD intercalates into DNA of dead or damaged cells, thus determination of 7-AAD positive cells is an indicator of cell viability. For 7-AAD tubes, the tubes were centrifuged at 400g for 5 min at 6C. After removing the supernatant, the cell pellets were resuspended in 100ul 1x 7-AAD solution. The tube was incubated on ice for 15 min in the dark. 1ml of staining buffer was added to the CD40L tube and then the 3 tubes were centrifuged together. After discarding the supernatant, the cell pellets were resuspended in 0.4ml staining buffer and FACS was run.

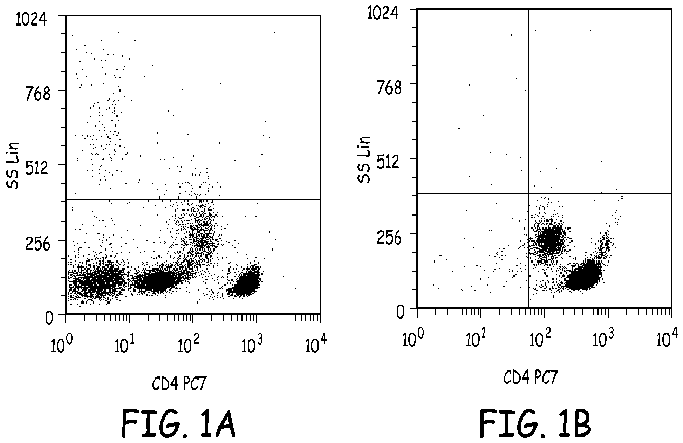

Example 1-Purification and cell phenotyping of CD4+ cells. Normal donor peripheral blood was obtained and the CD4+ cells from the buffy coat were purified using using Miltenyi magnetic beads and column obtained from Mitenyi Biotec (Auburn, CA). Fig. 1 is a flow cytometry plot of side scatter (measures size and density of cells) vs CD4 . Fig. 1 shows that CD4+ cells are about 46% of the total population (N=22). After positive selection for CD4+ cells, the result is 98.69% pure CD4+ cells (N=22). As can be seen from Fig. 1B, the population of CD4+ after the column contains both lymphocytes and monocytes.

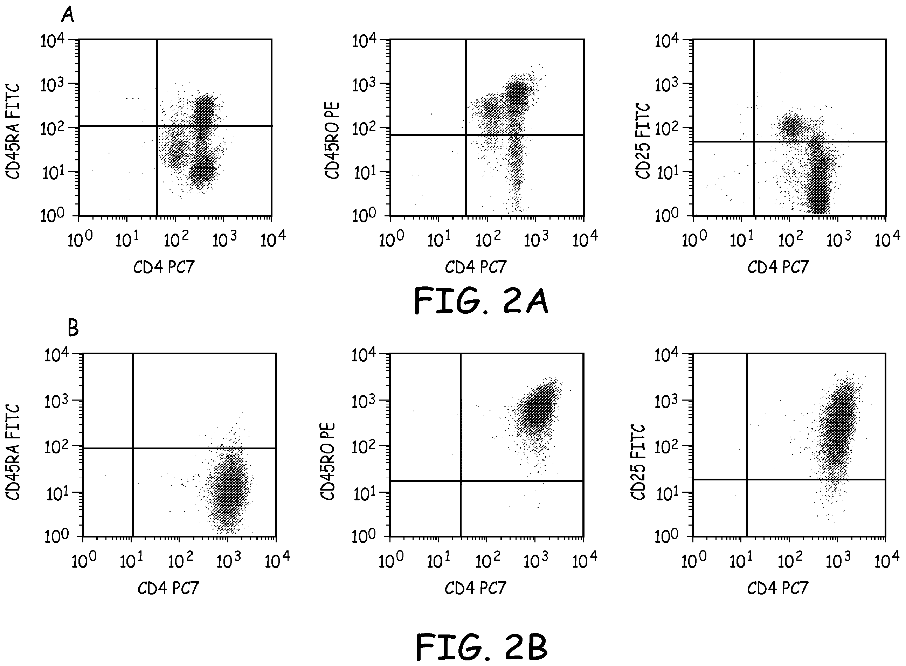

Since every batch is produced from a different donor, antigen markers were tested at the beginning and the end of production. Cell phenotyping of CD4+ cells (Fig. 2A) versus Cells activated in culture (CAC) (Fig. 2B) were tested. The results show a very consistent shift from a naive CD4+ phenotype (CD45RA+) to a memory phenotype (CD45RO+). Fig. 2A shows the phenotypes of the CD4+ cells for CD45RA, CD45RO and CD25, respectively. Fig. 2B shows the phenotypes of the CAC after 9 days of culturing and analysis for surface expression of CD45RA, CD45RO and CD25, respectively. From a mixed CD45RA/CD45RO population, a CD45RA negative CD45RO positive population is obtained. CD25+ cells are approximately 10% at the beginning of the expansion process. At day 9, CD25+ was approximately 85%. This is in accordance with the activation state of the cells. Fig. 2A shows CD4+ cells after column show a naive phenotype of CD45RA + cells (avg=44.76% n=29), CD45RO+ (avg= 53.44% n=29) and CD25+ (avg= 10.83% n=29). Fig. 2B shows that after 9 days in culture with continued stimulation, the phenotype changes into Th memory like cells. CD45RA goes down drastically (avg= 4.22% n=29), CD45RO+ goes up to avg of 97.8 (n=29), and CD25+ also goes up to average of 90.03% (n=29).

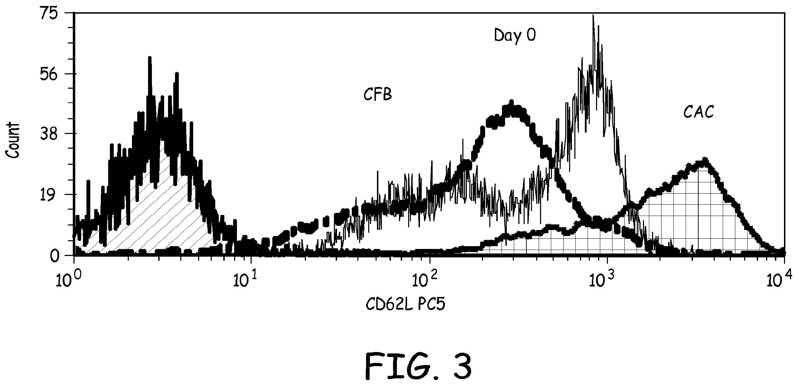

CD62L Expression pattern -Fig. 3 shows that at Day 0, CD4+ seed cells express a bimodal CD62L mean fluorescent intensity (MFI) with a maximum peak at approximately 10³ (the average arithmetic mean is 541 n=17). The MFI increases after 9 days in culture to an average arithmetic mean of 1541 (CAC) and then becomes significantly lower (average arithmetic mean of 190.3) after the final 4 hours activation (CFB).

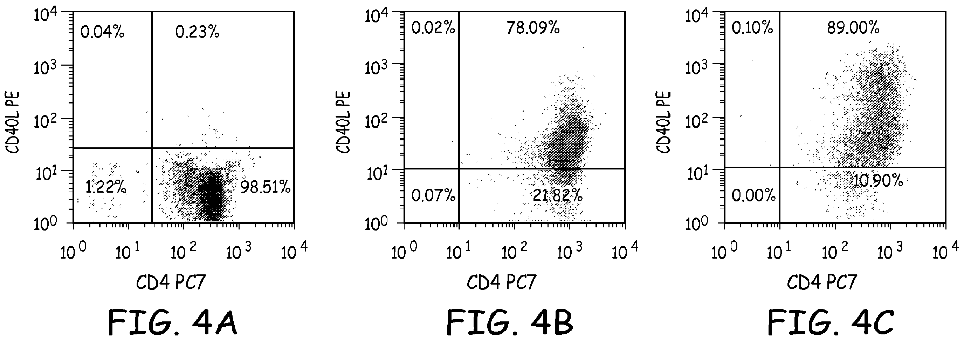

CD40L Expression Pattern--The density of CD40L expression on CAC was consistently increased on the surface of CAC after a 4 hour activation with CD3/CD28 beads. Fig. 4 shows the change in the arithmetic mean and percentage of CD40L expression of a representative batch comparing CD4+ at day 0 (Fig. 4A), day 9 harvested CAC before final activation (Fig. 4B) and after 4 hours activation, CFB (Fig. 4C). As can be seen, the difference between the un-activated and activated cells is not so much in the percent of CD40L+ cells, but rather in the arithmetic mean (the average AM for CAC is 20 (n=29) while the average for CFB is 112 (n=29)

Cytokine secretion during final activation-Samples of 9 day harvested CAC cells from each production batch were activated with CD3/CD28 beads for 4 hours and the supernatants were collected and tested for cytokine production (IFNγ, IL-4,IL-8, TNFα and GM-CSF) using ELISA assay. Table 1 shows the results of these experiments.

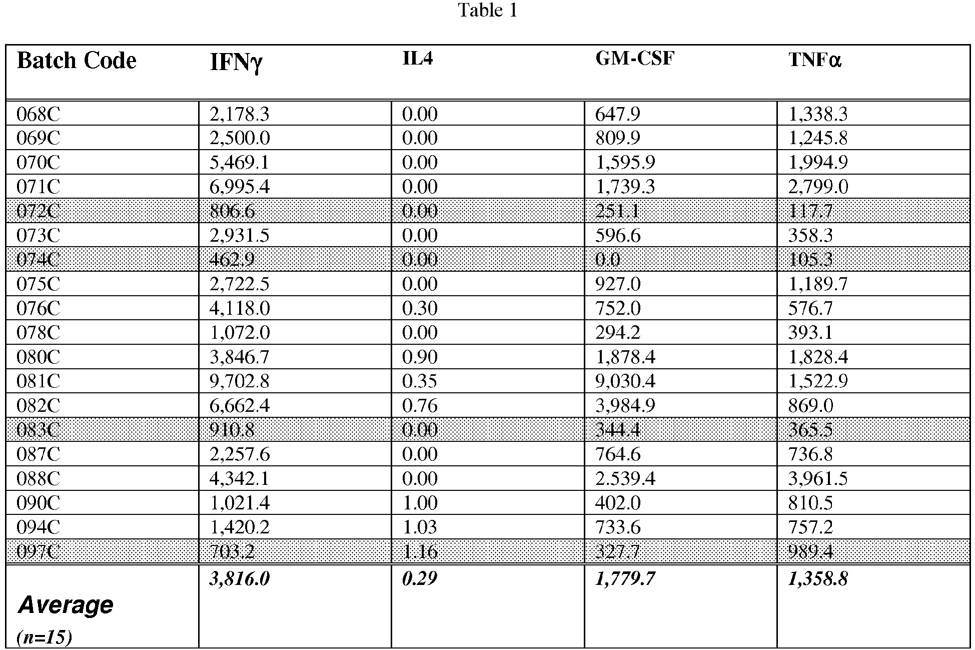

Only IFN © and IL-4 values were used for functional release criteria. The results for 19 consecutive batches are shown Table 1 (values are expressed as pg/10⁶ cells/4 hours). The reported average includes only passing batches.

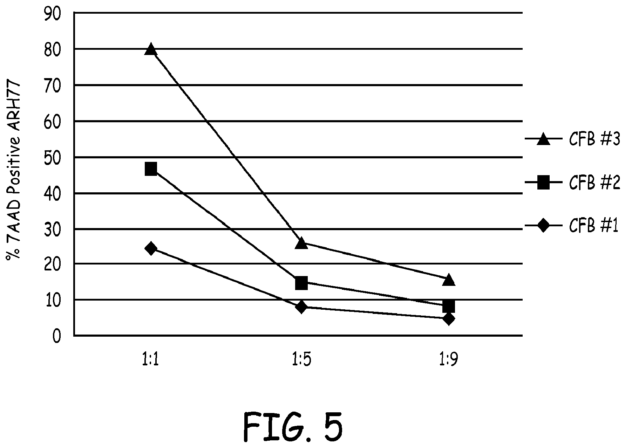

Example 2-Direct Killing by activated Th1/killer cells. To test if CFB may have direct effect on tumor cells, the ability of the cells to kill the ARH77 cell line was tested. This cell line is an established myeloma cell line. The ARH77 cells were stained in CFSE to differentiate them from the CFB. CFB were mixed with the stained ARH77 cells at different Effector (CFB) to Target (ARH77) for 18 hours. After that time, 7AAD was added and cells were acquired using the FC500MPL flow cytometer. This experiment was repeated with different CAC batches and with fresh and thawed CFB. ARH77 alone, with DynaBeads, CD4+ cells or with CAC had no significant death (less than 10%) data not shown. The results shown in Fig. 5 indicate that CFB, but not CAC, CD4+ cells or beads, have direct killing effect on the ARH77 cell line.

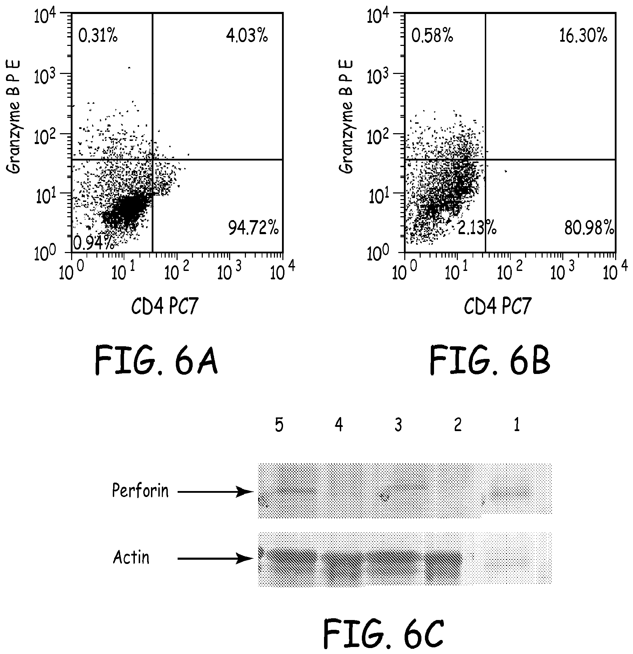

The nature of the direct killing effect was examined to see if the effect was due to the Perforin-Granzyme pathway. Both internal staining and western blot techniques were used to stain for Perforin and Granzyme B. Fig. 6A shows that CAC express very little Granzyme B, as can be seen. Fig. 6B shows that CFB express significant amount of Granzyme B. Fig. 6C shows that Perforin can be detected only by the use of western blot. Lane 1 is the extract of activated PBMCs, as a positive control. Lane 2&4 are of CAC from different batches, and lanes 3&5 are of the CFB from the same batches.

Inhibition assays were also performed to elucidate the nature of the direct killing mechanism. CFB were mixed with ARH77 cells as described above and different inhibitory agents were added as shown below Table 2. Antibodies against CD40L and/or FAS-L were added to the CFB:ARH77 mix. Table 2 shows the even the highest concentration did not prevent the killing profile.

**Table 2**

| | **Anti C40L** | **no inhibition** | **0.1ìg/ml** | **1ìg/ml** | **20ìg/ml** |
|---|---|---|---|---|---|
| **Anti Fas-L** | | | 35.48 | 35.92 | 35.5 |
| **no inhibition** | | 33.85 | | | |
| **1ng**/**ml** | 36.55 | | 34.55 | | |
| **10ng**/**ml** | 38.86 | | | | |
| **200ng**/**ml** | 38.97 | | | | 30.0 |

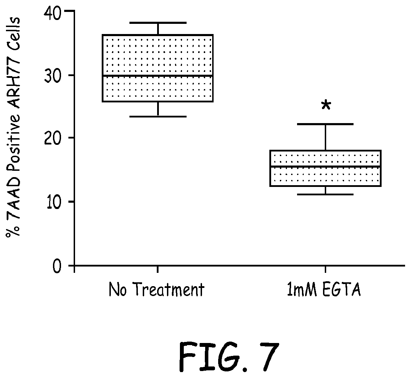

The effect of EGTA on the direct killing mechanism was examined. EGTA blocks the polymerization of Perforin and acts as an inhibitor of the Perforin/Granzyme B mechanism. 1mM EGTA was added to the direct killing assay described above. As shown in Fig. 7, 1mM EGTA reduced the killing by an average of 50% (n=7) Paired t test P value 0.0002. The direct killing effect that CFB show comes from the Perforin-Granzyme pathway.

## Claims

1. A composition comprising activated CD4+ Thl/killer cells having Th1 characteristics and direct cytolytic activity against a tumour cell line, wherein the Thl/killer cells are *ex vivo* activated CD4+ cells expressing granzyme B and perforin obtained by activating naive CD4+ cells by cross-linking of CD3 and CD28 cell surface molecules without the addition of exogenous cytokines, freezing the activated cells and then reactivating the cells by cross-linking of CD3 and CD28 cell surface molecules.

2. The composition of claim 1 wherein the cytolytic activity comprises NK characteristics.

3. The composition of claim 1 wherein the Th1/killer cells express IFN-gamma.

4. The composition of claim 1 wherein the Th1/killer cells have substantially reduced or no expression of IL-4.

5. The composition of claim 1 wherein the Th1/killer cells are derived from healthy donor peripheral blood.

6. The composition of claim 1 wherein the composition comprises at least about 1 x 10⁷ cells.

7. The composition of claim 1 wherein the composition comprises at least about 1 x 10⁸ cells.

8. The composition of claim 1 wherein the cytolytic activity of the Thl/killer cells specifically inactivates diseased cells and not cells obtained from healthy donors, and wherein the diseased cells preferably comprise cancerous cells, infected cells or combinations thereof.

9. The composition of claim 1 wherein the tumor cell line is ARH77.

10. An *in vitro* method for destroying diseased cells comprising contacting the diseased cells with a composition according to any one of claims 1 to 9 wherein the interaction of the diseased cells directly with the activated CD4+ Th1/killer cells of the composition leads to destruction of the diseased cells.

11. The method of claim 10 wherein the diseased cells comprise cancerous cells, infected cells or a combination thereof.

12. A composition comprising activated CD4+ Th1/killer cells according to any one of claims 1 to 9 for use in treating a patient, wherein the composition comprises an effective number of the Thl/killer cells.

13. The composition for use according to claim 12 wherein the Th1/killer cells are allogeneic to the patient.

## Patentansprüche

1. Zusammensetzung, umfassend aktivierte CD+4 Th1/Killerzellen mit Th1-Charakteristiken und einer direkten zytolytischen Aktivität gegen eine Tumorzelllinie, wobei die Th1/Killerzellen *ex vivo* aktivierte CD4+ Zellen zur Expression von Granzyme B und Perforin sind, erhalten durch die Aktivierung von naiven CD4+ Zellen durch das Vernetzen von CD3- und CD28-Zelloberflächenmolekülen ohne die Zugabe von exogenen Zytokinen, das Einfrieren der aktivierten Zellen und das anschließende Reaktivieren der Zellen durch das Vernetzen von CD3- und CD28-Zelloberflächenmolekülen.

2. Zusammensetzung nach Anspruch 1, wobei die zytolytische Aktivität NK-Charakteristiken umfasst.

3. Zusammensetzung nach Anspruch 1, wobei die Th1/Killerzellen IFN-gamma exprimieren.

4. Zusammensetzung nach Anspruch 1, wobei die THh1/Killerzellen eine im Wesentlichen reduzierte oder keine Expression von IL-4 aufweisen.

5. Zusammensetzung nach Anspruch 1, wobei die Th1/Killerzellen von peripherem Blut eines gesunden Spenders stammen.

6. Zusammensetzung nach Anspruch 1, wobei die Zusammensetzung mindestens etwa 1 x 10⁷ Zellen enthält.

7. Zusammensetzung nach Anspruch 1, wobei die Zusammensetzung mindestens etwa 1 x 10⁸ Zellen enthält.

8. Zusammensetzung nach Anspruch 1, wobei die zytolytische Aktivität der Th1/Killerzellen speziell erkrankte Zellen inaktiviert und nicht Zellen, die von gesunden Spendern stammen, und wobei die erkrankten Zellen bevorzugt kanzeröse Zellen, infizierte Zellen oder Kombinationen derselben umfassen.

9. Zusammensetzung nach Anspruch 1, wobei die Tumorzelllinie ARH77 ist.

10. *In*-*vitr*o-Verfahren zum Zerstören von erkrankten Zellen, umfassend das Inkontaktbringen der erkrankten Zellen mit einer Zusammensetzung gemäß einem der Ansprüche 1 bis 9, wobei die Interaktion der erkrankten Zellen direkt mit den aktivierten CD4+ Th1/Killerzellen der Zusammensetzung zu einer Zerstörung der erkrankten Zellen führt.

11. Verfahren nach Anspruch 10, wobei die erkrankten Zellen kanzeröse Zellen, infizierte Zellen oder eine Kombination derselben umfassen.

12. Zusammensetzung, enthaltend aktivierte CD4+ Th1/Killerzellen gemäß einem der Ansprüche 1 bis 9 zur Verwendung bei der Behandlung eines Patienten, wobei die Zusammensetzung eine wirksame Anzahl von Th1/Killerzellen enthält.

13. Zusammensetzung zur Verwendung gemäß Anspruch 12, wobei die Th1/Killerzellen für den Patienten allogen sind.

## Revendications

1. Composition comprenant :
des cellules CD4+ Th1 tueuses activées ayant des caractéristiques Th1 et exerçant une activité cytolytique directe contre une lignée de cellules tumorales, les cellules Th1 tueuses étant des cellules CD4+ activées *ex vivo* exprimant un granzyme B et une perforine qui sont obtenues en activant des cellules CD4+ naïves par réticulation de molécules de surface de cellules CD3 et CD28 sans ajout de cytokines exogènes, congélation des cellules activées, puis réactivation des cellules par réticulation de molécules de surface de cellules CD3 et CD28.

2. Composition suivant la revendication 1, dans laquelle l'activité cytolytique comprend des caractéristiques NK.

3. Composition suivant la revendication 1, dans laquelle les cellules Th1 tueuses expriment un IFN-gamma.

4. Composition suivant la revendication 1, dans laquelle les cellules Th1 tueuses présentent une expression essentiellement réduite ou pas d'expression du tout d'IL-4.

5. Composition suivant la revendication 1, dans laquelle les cellules Th1 tueuses sont dérivées du sang périphérique d'un donneur sain.

6. Composition suivant la revendication 1, la composition comprenant au moins 1 x 10⁷ cellules environ.

7. Composition suivant la revendication 1, la composition comprenant au moins 1 x 10⁸ cellules environ.

8. Composition suivant la revendication 1, dans laquelle l'activité cytolytique des cellules Th1 tueuses inactive spécifiquement des cellules malades et non des cellules obtenues de donneurs sains et dans laquelle les cellules malades comprennent de préférence des cellules cancéreuses, des cellules infectées ou des combinaisons de celles-ci.

9. Composition suivant la revendication 1, dans laquelle la lignée de cellules tumorales est ARH77.

10. Procédé de destruction de cellules malades *in vitro* comprenant la mise en contact des cellules malades avec une composition suivant une quelconque des revendications 1 à 9, dans lequel l'interaction directe des cellules malades avec les cellules CD4+ Th1 tueuses activées de la composition entraîne la destruction des cellules malades.

11. Procédé suivant la revendication 10, dans lequel les cellules malades comprennent des cellules cancéreuses, des cellules infectées ou une combinaison de celles-ci.

12. Composition comprenant des cellules CD4+ Th1 tueuses activées suivant une quelconque des revendications 1 à 9 destinée à être utilisée dans le traitement d'un patient, la composition comprenant un nombre effectif de cellules Th1 tueuses.

13. Composition destinée à une utilisation suivant la revendication 12, dans laquelle les cellules Th1 tueuses sont allogéniques au patient.
